# EUROPÄISCHE PATENTSCHRIFT

(11) **EP 0 870 494 B1**
(45) Veröffentlichungstag und Bekanntmachung des Hinweises auf die Patenterteilung: **04.06.2003**
(21) Anmeldenummer: 98105287.1
(22) Anmeldetag: 24.03.1998
(51) Int. Cl.: A61K 7/50

(54) **Kosmetische und dermatologische waschaktive Zubereitungen, enthaltend Acrylatcopolymere, Alkylglucoside und Alkohole**
Cosmetic and dermatological washing composition containing acrylate copolymers, alkylglucosides and alcohols
Composition détergente cosmétique et dermatologique contenant des copolymères d'acrylates des alkylglucosides et des alcools

(30) Priorität: 08.04.1997 DE 19714424
(43) Veröffentlichungstag der Anmeldung: 14.10.1998
(73) Patentinhaber: Beiersdorf Aktiengesellschaft, 20245 Hamburg (DE)
(72) Erfinder: Lerg, Heike, 22303 Hamburg (DE); Schmucker, Robert, Dr., 22523 Hamburg (DE)

(56) Entgegenhaltungen:
- EP-A- 0 422 862
- WO-A-98/47474
- FR-A- 2 731 616
- US-A- 4 678 595
- CHEMICAL ABSTRACTS, vol. 123, no. 6, 7. August 1995 (1995-08-07) Columbus, Ohio, US; abstract no. 65547, ARAI, YASUHIRO ET AL: "Acidic hair dye compositions containing carboxyvinyl polymer and alcohols with high adhesion and compatibility to hair" XP002121407 & JP 07 101841 A (SHISEIDO CO LTD, JAPAN) 18. April 1995 (1995-04-18)
- CHEMICAL ABSTRACTS, vol. 117, no. 18, 2. November 1992 (1992-11-02) Columbus, Ohio, US; abstract no. 173836, TOSAKA, MASAKI ET AL: "Liquid toilet cleaning compositions with good storability" XP002121408 & JP 04 096999 A (KAO K. K., JAPAN) 30. März 1992 (1992-03-30)
- DATABASE WPI Section Ch, Week 198938 Derwent Publications Ltd., London, GB; Class A25, AN 1989-276086 XP002121409 & JP 01 203036 A (SHISEIDO CO LTD), 15. August 1989 (1989-08-15)
- DATABASE WPI Section Ch, Week 199220 Derwent Publications Ltd., London, GB; Class A97, AN 1992-163827 XP002121410 & JP 04 103696 A (KAO CORP), 6. April 1992 (1992-04-06)

## Beschreibung

Die vorliegende Erfindung betrifft kosmetische Reinigungsmittel. Derartige Mittel sind an sich bekannt. Es handelt sich dabei im wesentlichen um oberflächenaktive Substanzen oder Stoffgemische, die dem Verbraucher in verschiedenen Zubereitungen angeboten werden.

Zubereitungen dieser Art sind beispielsweise Schaum- und Duschbäder, feste und flüssige Seifen oder sogenannte "Syndets" (synthetische Detergentien), Shampoos, Handwaschpasten, Intimwaschmittel, spezielle Reinigungsmittel für Kleinkinder und dergleichen.

Oberflächenaktive Stoffe - am bekanntesten die Alkalisalze der höheren Fettsäuren, also die klassischen "Seifen" - sind amphiphile Stoffe, die organische unpolare Substanzen in Wasser emulgieren können.

Diese Stoffe schwemmen nicht nur Schmutz von Haut und Haaren, sie reizen, je nach Wahl des Tensids oder des Tensidgemisches, Haut und Schleimhäute mehr oder minder stark.

Das gebräuchlichste Tensid für kosmetische Zusammensetzungen ist das Natriumlaurylethersulfat. Obwohl es gute Waschkraft besitzt und gut haut- und schleimhautverträglich ist, sollten empfindliche Personen den häufigen Kontakt damit meiden.

Es ist zwar eine große Zahl recht milder Tenside erhältlich, jedoch sind die Tenside des Standes der Technik entweder mild, reinigen aber schlecht, oder aber sie reinigen gut, reizen jedoch Haut oder Schleimhäute.

Diesem Übelstande galt es also, Abhilfe zu schaffen.

Die vorliegende Erfindung betrifft in einer besonderen Ausführungsform Reinigungszubereitungen für die Verwendung als Duschpräparat.

Auch derartige Zubereitungen sind an sich bekannt. Es handelt sich dabei im wesentlichen um oberflächenaktive Substanzen oder Stoffgemische, die dem Verbraucher in verschiedenen Zubereitungen angeboten werden. Zubereitungen solcher Art zeichnen sich im allgemeinen durch einen mehr oder weniger hohen Wassergehalt aus, können aber auch beispielsweise als Konzentrat vorliegen.

Im allgemeinen unterscheiden sich Präparate, welche für das Duschbad vorgesehen sind, nicht oder kaum von Wannenbadzubereitungen, abgesehen davon, daß bei Duschzubereitungen Produkte höherer Viskosität bevorzugt werden, die nicht nach Entnahme aus dem Behälter aus der Hand rinnen. Dies ist bei Wannenbadzubereitungen weniger von praktischer Bedeutung.

Schon bei einem einfachen Wasserbade ohne Zusatz von Tensiden kommt es zunächst zu einer Quellung der Homschicht der Haut, wobei der Grad dieser Quellung beispielsweise von der Dauer des Bades und dessen Temperatur abhängt. Zugleich werden wasserlösliche Stoffe, z.B. wasserlösliche Schmutzbestandteile, aber auch hauteigene Stoffe, die für das Wasserbindungsvermögen der Homschicht verantwortlich sind, ab- bzw. ausgewaschen. Durch hauteigene oberflächenaktive Stoffe werden zudem auch Hautfette in gewissem Ausmaße gelöst und ausgewaschen. Dies bedingt nach anfänglicher Quellung eine nachfolgende deutliche Austrocknung der Haut, die durch waschaktive Zusätze nach verstärkt werden kann.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Falle nichtpathologischer Abweichungen vom Normalstatus, z.B. durch umweltbedingte Abnutzungsschäden bzw. Irritationen, Lichtschäden, Altershaut usw., ist der Schutzmechanismus der Hautoberfläche gestört. Unter Umständen ist er dann aus eigener Kraft nicht mehr imstande, seine Aufgabe zu erfüllen und muß durch externe Maßnahmen regeneriert werden.

Aufgabe der vorliegenden Erfindung war somit, diesem Mangel des Standes der Technik Abhilfe zu schaffen. Weiterhin war eine Aufgabe der Erfindung, Wannenaber auch Duschbadzubereitungen zur Verfügung zu stellen, welche einesteils hohe Pflegewirkung besitzen, ohne daß andererseits die reinigende Wirkung dahinter zurücksteht.

Die vorliegende Erfindung betrifft ferner waschaktive haarkosmetische Zubereitungen, landläufig als Shampoos bezeichnet. Insbesondere betrifft die vorliegende Erfindung haarkosmetische Wirkstoffkombinationen und Zubereitungen zur Pflege des Haars und der Kopfhaut.

Auch die Haarwäsche mit aggressiven Tensiden kann das Haar beanspruchen, zumindest dessen Erscheinungsbild oder das Erscheinungsbild der Haartracht insgesamt herabsetzen. Beispielsweise können bestimmte wasserlösliche Haarbestandteile (z.B. Harnstoff, Harnsäure, Xanthin, Keratin, Glycogen, Citronensäure, Milchsäure) durch die Haarwäsche herausgelaugt werden.

Der Stande der Technik ließ es aber an Shampooformulierungen mangeln, welche dem geschädigten Haar in befriedigender Weise Pflege zukommen ließen. Aufgabe war daher, auch diesen Nachteilen des Standes der Technik Abhilfe zu schaffen.

Übliche, und sich gerade in neuerer Zeit immer weiter verbreitende kosmetische und dermatologische Zubereitungsformen sind Gele.

Kosmetische Gele erfreuen sich beim Verbraucher äußerster Beliebtheit. Da sie meistens durchsichtig sind, oftmals eingefärbt aber ebensooft farblos klar sein dürften, bieten sie dem kosmetischen Entwickler zusätzliche Gestaitungsmöglichkeiten, die teilweise funktionalen Charakter haben, teilweise aber auch lediglich der Aufbesserung des äußeren Erscheinungsbildes dienen. So können beispielsweise dem Produkt, welches sich dem Betrachter dann in der Regel in einer durchsichtigen Verpackung darbietet, durch eingearbeitete Farbpigmente, Gasbläschen und dergleichen, oder aber auch größere Objekte, interessante optische Effekte verliehen werden.

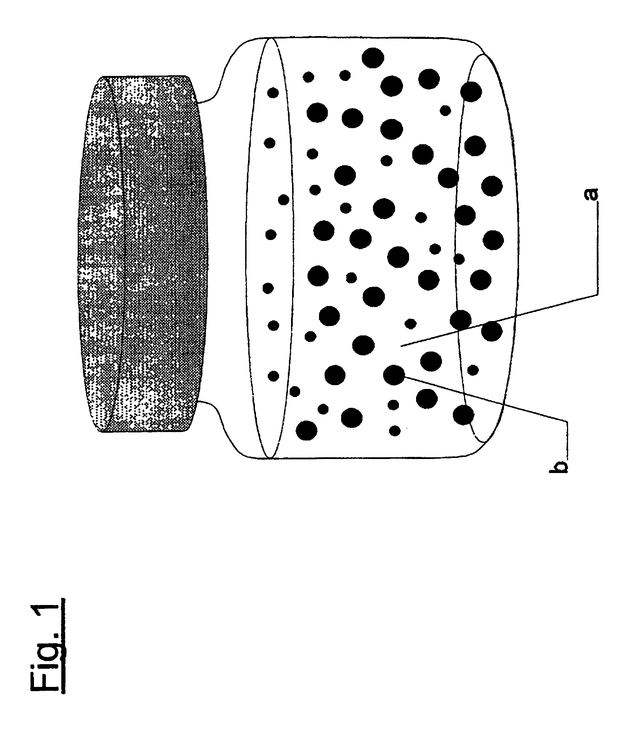

Gerade dann, wenn es erwünscht ist, daß das oder die eingearbeiteten Objekte, mögen diese als solche mit dem bloßen Auge als solche erkenntlich sein, mögen sie in mikroskopischen Ausmaßen, aber in interessanter Anordnung - beispielsweise in Form von künstlich erzeugten Farbschlieren - dann doch sichtbare Formen ergeben, so ist es doch wünschenswert, daß diese Objekte in der Gelformulierung ortsfest bleiben und nicht zu Boden sinken oder in irgendeiner Weise in der Formulierungen andere unliebsame Wanderungen vornehmen. Dies ist in Fig. 1 abgebildet, in der das Bezugszeichen a auf ein im wesentlich klares Gel verweist, In welches diskrete, mit dem bloßen Auge erkenntliche Partikel b eingearbeitet sind.

Flüssigkeiten können bezüglich ihrer rheologischen Eigenschaften durch ihr Fließund Deformationsverhalten unterschieden werden. Ideal elastische Körper erleiden durch äußere Kräfte eine elastische Deformation, die bei Wegnahme der äußeren Krafteinwirkung ein spontanes, vollständiges Zurückgehen der Deformation bewirkt. Ideal viskose Körper werden durch äußere Kräfte irreversibel in ihrer Form verändert. Die zunehmende Deformation wird als Fließen bezeichnet. Die meisten Flüssigkeiten sind weder ideal viskos noch ideal elastisch, sondern zeigen sowohl viskose als auch elastische Eigenschaften und werden daher als viskoelastische Substanzen bezeichnet.

Im Großteil viskoelastischer Lösungen werden dispergierte Partikel oder Gasbläschen immer sedimentieren bzw. aufsteigen. Sie besitzen eine endliche Strukturrelaxationszeit. Das bedeutet, daß die Netzwerke in diesen Systemen auf eine Deformation mit einer entsprechenden Schubspannung reagieren. Diese wird aber in einer endlichen Zeit auf den Wert Null relaxieren, so daß sich die gesamte Lösung wieder in einem stabilen Ruhezustand ohne Spannung befindet. Dies bedeutet weiter, daß diese Lösungen eine definierte Nullviskosität besitzen und somit bei kleinen Scherraten einen konstanten Viskositätswert erreichen.

Im Gegensatz zu diesen Systemen gibt es aber auch solche, in denen dispergierte Partikel oder Gasbläschen nicht sedimentieren. Es fällt auf, daß diese Systeme erst oberhalb eines charakteristischen Werts fließen. Dieser Wert wird Fließgrenze genannt. Bei näherer Betrachtung der rheologischen Eigenschaften dieser Systeme fällt auf, daß der Speichermodul in ganzen Frequenzbereich unabhängig ist von der Oszillationsfrequenz und immer wesentlich größer ist als der Verlustmodul.

Dagegen erreicht der Betrag der komplexen Viskosität auch bei den kleinsten Frequenzen keinen konstanten Wert, sondern steigt weiter an.

Carbopolgele sind quervemetzte Acrylsäurepolymere, die eine hohe Anzahl von Carboxylgruppen tragen. In gelöster Form binden diese Strukturen Wasser. Die Neutralisation der Carboxylgruppen führt aufgrund deren elektrostatischen Abstoßung zu einer Ausdehnung und damit Quellung der Polymerketten. In diesem Zustand erreichen die Carbopol Gele ihre typischen rheologischen Eigenschaften wie z.B. die Ausbildung einer Fließgrenze.

Der Effekt der Ausbildung einer Fließgrenze beruht somit auf der elektrostatischen Abstoßung der Carboxylgruppen. Zusätzliche Elektrolyte schirmen diese Ladungen ab. Dadurch kollabieren die Netzwerke, die Fließgrenze bricht zusammen, Partikel oder Gasbläschen können nicht mehr in Schwebe gehalten werden.

Tenside wirken wie Elektrolyte. Daher war es bisher nicht möglich, gut schäumende Reinigungsprodukte mit einem entsprechend hohem Gehalt an Tensid zu formulieren, die klare Carbopol Gele mit Fließgrenze als Basis enthielten.

Der Stand der Technik kennt zwar bereits entsprechende Systeme mit Xanthan Gum (z.B. EP-A 738 509). Diese besitzen aber bezüglich des Hautgefühls während und nach der Anwendung schlechtere kosmetische Eigenschaften. Darüber hinaus können bei gleicher Einsatzkonzentration nur geringere Viskositäten erreicht werden. Die Ausgestaltung eines Gels, welche dazu geeignete Fließeigenschaften aufweist, bietet dem Fachmanne in der Regel keine überaus großen Schwierigkeiten, außer, wenn hohe Tensidkonzentrationen erreicht werden sollen - in der Regel eine Grundanforderung an Reinigungsprodukte. Der Nachteil solch hoher Tensidkonzentrationen ist, daß meistens nur eingetrübte, trübe oder gar opake Produkte erlangt werden.

Auch diesem Nachteil des Standes der Technik galt es also, Abhilfe zu schaffen.

Es hat sich überraschend gezeigt, und darin liegt die Lösung dieser Aufgaben, daß kosmetische und dermatologische waschaktive Zubereitungen, enthaltend
(a) eine wirksame Menge einer oder mehrerer grenzflächenaktiven Substanzen, gewählt aus der Gruppe der Alkylglucoside, welche sich durch die Strukturformel 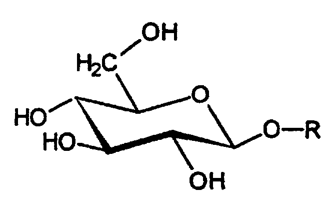 auszeichnen, wobei R einen verzweigten oder unverzweigten Alkylrest mit 1 bis 24 Kohlenstoffatomen darstellt,
(b) eine wirksame Menge eines oder mehrerer gelbildender Acrylat-Alkylacrylat-Copolymere und
(c) verzweigte und/oder unverzweigte aliphatische Alkohole mit 2 - 6 Kohlenstoffatomen und einer oder mehreren OH-Funktionen
den Nachteilen des Standes der Technik abhelfen.

Es war für den Fachmann daher nicht vorauszusehen gewesen, daß die erfindungsgemäßen Zubereitungen klare Gele mit hervorragenden rheologischen Eigenschaften bilden würden, die sich darüberhinaus auch noch in vorzüglicher Weise als waschaktive Substanzen eignen würden.

Vorteilhaft wird R in der Strukturformel des Alkylglucosids gewählt aus der Gruppe der unverzweigten Alkylreste, wobei der Hexyl-, der Heptyl-, der Octyl- der Nonylder Decyl-, der Undecyl-, der Dodecyl-, und der Tetradecylrest bevorzugt werden.

Die Gesamtmenge an einer oder mehreren erfindungsgemäß verwendeten grenzflächenaktiven Alkylglucosiden in den fertigen kosmetischen oder dermatologischen Zubereitungen wird vorteilhaft aus dem Bereich von 0,1 - 25,0 Gew.-%, bevorzugt 0,5 - 15,0 Gew.-% gewählt, bezogen auf das Gesamtgewicht der Zubereitungen.

Das oder die erfindungsgemäß vorteilhaft einzusetztende Acrylat-Alkylacrylat-Copolymere werden vorteilhaft gewählt aus der Gruppe der sogenannten Carbomere oder Carbopole (Carbopol®: eigentlich eingetragene Marke der Gesellschaft BFGoodrich Company). Insbesondere zeichnen sich das oder die erfindungsgemäß vorteilhaft einzusetzenden Acrylat-Alkylacrylat-Copolymere durch eine Struktur aus wie folgt:

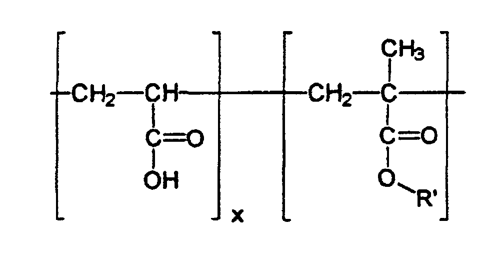

Dabei stellt R' einen langkettigen Alkylrest dar, x und y stellen Zahlen dar, welche den jeweiligen stöchiometrischen Anteil der jeweiligen Comonomere symbolisieren.

Beispielsweise vorteilhaft können als Acrylat-Alkylacrylat-Copolymere Produkte wie Carbopol® 1382 von der Gesellschaft BFGoodrich Company eingesetzt werden.

Die Gesamtmenge an einer oder mehreren erfindungsgemäß verwendeten grenzflächenaktiven Acrylat-Alkylacrylat-Copolymeren in den fertigen kosmetischen oder dermatologischen Zubereitungen wird vorteilhaft aus dem Bereich von 0,1 - 10,0 Gew.-%, bevorzugt 0,5 - 2,5 Gew.-% gewählt, bezogen auf das Gesamtgewicht der Zubereitungen.

Erfindungsgemäß werden die Alkohole bevorzugt gewählt aus der Gruppe Ethanol, Ethylenglycol, Propylenglycol, Glycerin, Isopropylalkohol, 1,3-Butylenglycol, 2,3-Butylenglycol.

Die erfindungsgemäßen Zubereitungen sind besonders bevorzugt dadurch gekennzeichnet, daß die Gesamtmenge an einer oder mehreren erfindungsgemäß verwendeten Alkoholen in den fertigen kosmetischen oder dermatologischen Zubereitungen aus dem Bereich von 0,1 - 25,0 Gew.-%, bevorzugt 0,5 - 15,0 Gew.-% gewählt wird, bezogen auf das Gesamtgewicht der Zubereitungen.

Die erfindungsgemäßen waschaktiven Zubereitungen zeichnen sich in der Regel durch einen Wassergehalt von 95 - 5 Gew.-% aus, bezogen auf das Gesamtgewicht der Zubereitungen und stellen Gele dar.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Camosin, D-Carnosin, L-Carnosin und deren Derivate (z.B. Anserin), Carotinoide, Carotine (z.B. α-Carotin. β-Carotin, ψ-Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butylund Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), femer (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure. Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1-10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Herstellung der erfindungsgemäßen kosmetischen und/oder dermatologischen Zubereitungen erfolgt in der dem Fachmann üblichen Weise, zumeist dergestallt, daß die erfindungsgemäß verwendeten grenzflächenaktiven Glucosederivaten bei gleichmäßigem Rühren und gegebenenfalls unter Erwärmen suspendiert und gewünschtenfalls homogenisiert, gegebenenfalls mit weiteren Lipidkomponenten und gegebenenfalls mit einem oder mehreren weiteren Emulgatoren vereinigt, hernach die Ölphase mit der wäßrigen Phase, in welche gegebenenfalls ein Verdickungsmittel eingearbeitet worden ist, und welche vorzugsweise etwa die gleiche Temperatur besitzt wie die Ölphase, vermischt, gewünschtenfalls homogenisiert und auf Raumtemperatur abkühlen läßt. Nach Abkühlen auf Raumtemperatur kann, insbesondere, wenn noch flüchtige Bestandteile eingearbeitet werden sollen, nochmaliges Homogenisieren erfolgen.

Die erfindungsgemäßen Zubereitungen sind besonders vorteilhaft dadurch gekennzeichnet, daß gasförmige, feste und oder flüssige Objekte In die Gele eingebettet vorliegen. Dem Fachmann ist dabei bekannt, wie die Einarbeitung solcher Objekte in die Zubereitung vonstatten geht.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel 1

| | Gew.-% |
|---|---|
| Decylglucosid (50%) | 20,00 |
| Carbopol® 1382 | 1,50 |
| Natriumhydroxid | 0,75 |
| Butylenglycol | 10,00 |
| Propylenglycol | 17,50 |
| ZnSO₄ | 0,01 |
| Süßholzwurzelextrakt | 0,10 |
| Calcium Pantothenat | 0,10 |
| Na₃HEDTA | 0,50 |
| Kamillenextrakt | 0,10 |
| Parfum, Konservierungsmittel | q.s. |
| Wasser | ad 100,00 |

### Beispiel 2

| | Gew.-% |
|---|---|
| Decylgtucosid (50%) | 20,00 |
| Carbopol® 1382 | 1,25 |
| Natriumhydroxid | 0,63 |
| Butylenglycol | 10,00 |
| Propylenglycol | 17,50 |
| ZnSO₄ | 0,01 |
| Sußholzwurzelextrakt | 0,10 |
| Calcium Pantothenat | 0,10 |
| Na₃HEDTA | 0,50 |
| Kamillenextrakt | 0,10 |
| Parfum, Konservierungsmittel | q.s. |
| Wasser | ad 100,00 |

### Beispiel 3

| | Gew.-% |
|---|---|
| Decylglucosid (50%) | 20,00 |
| Carbopol® 1382 | 1,25 |
| Natriumhydroxid | 0,65 |
| Butylenglycol | 10.00 |
| Ethanol | 11.00 |
| Na₃HEDTA | 1.00 |
| Parfum, Konservierungsmittel | q.s. |
| Wasser | ad 100,00 |

### Beispiel 4

| | Gew.-% |
|---|---|
| Decylglucosid (50%) | 10.00 |
| Carbopol® 1382 | 0.75 |
| Natriumhydroxid | 0.35 |
| Butylenglycol | 10.00 |
| Propylenglycol | 11.00 |
| Na₃HEDTA | 0.50 |
| Parfum, Konservierungsmittel | q.s. |
| Wasser | ad 100,00 |

### Beispiel 5

| | Gew.-% |
|---|---|
| Decylglucosid (50%) | 20,00 |
| Carbopol® 1382 | 1,00 |
| Natriumhydroxid | 0.50 |
| Butylenglycol | 10,00 |
| Propylenglycol | 17,50 |
| Na₃HEDTA | 0,50 |
| Parfum, Konservierungsmittel | q.s. |
| Wasser | ad 100,00 |

### Beispiel 6

| | Gew.-% |
|---|---|
| Decylglucosid (50%) | 30,00 |
| Carbopol® 1382 | 1,25 |
| Natriumhydroxid | 0,50 |
| Butylenglycol | 5,00 |
| Propylenglycol | 17,50 |
| Na₃HEDTA | 0,50 |
| Parfum, Konservierungsmittel | q.s. |
| Wasser | ad 100,00 |

## Patentansprüche

1. Kosmetische und dermatologische waschaktive Zubereitungen, enthaltend:
(a) eine wirksame Menge einer oder mehrerer grenzflächenaktiven Substanzen, gewählt aus der Gruppe der Alkylglucoside, welche sich durch die Strukturformel 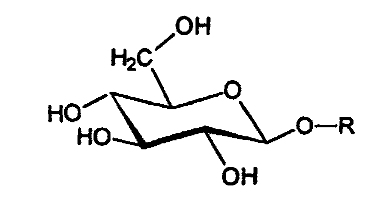 auszeichnen, wobei R einen verzweigten oder unverzweigten Alkylrest mit 1 bis 24 Kohlenstoffatomen darstellt,
(b) eine wirksame Menge eines oder mehrerer gelbildender Acrylat-Alkylacrylat-Copolymere und
(c) verzweigte und/oder unverzweigte aliphatische Alkohole mit 2 - 6 Kohlenstoffatomen und einer oder mehreren OH-Funktionen.

2. Zubereitungen nach Anspruch 1, **dadurch gekennzeichnet, daß** R in der Strukturformel des Alkylglucosids gewählt wird aus der Gruppe der unverzweigten Alkylreste, wobei der Hexyl-, der Heptyl-, der Octyl- der Nonyl- der Decyl-, der Undecyl-, der Dodecyl-, und der Tetradecylrest bevorzugt werden.

3. Zubereitungen nach Anspruch 1, **dadurch gekennzeichnet, daß** die Gesamtmenge an einer oder mehreren erfindungsgemäß verwendeten grenzflächenaktiven Alkylglucosiden in den fertigen kosmetischen oder dermatologischen Zubereitungen aus dem Bereich von 0,1 - 25,0 Gew.-%, bevorzugt 0,5 - 15,0 Gew.-% gewählt wird, bezogen auf das Gesamtgewicht der Zubereitungen.

4. Zubereitungen nach Anspruch 1, **dadurch gekennzeichnet, daß** das oder die Acrylat-Alkylacrylat-Copolymere gewählt werden aus der Gruppe der sogenannten Carbomere, insbesondere gewählt aus der Gruppe der Carbomere der Struktur: 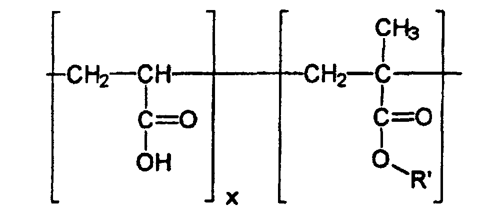 wobei R' einen langkettigen Alkylrest dar stellt, x und y Zahlen dar stellen, welche den jeweiligen stöchiometrischen Anteil der jeweiligen Comonomere symbolisieren.

5. Zubereitungen nach Anspruch 1, **dadurch gekennzeichnet, daß** die Alkohole gewählt werden aus der Gruppe Ethylenglycol, Propylenglycol, Glycerin, Isopropylalkohol, 1,3-Butylenglycol, 2,3-Butylenglycol, Ethanol.

6. Zubereitungen nach Anspruch 1, **dadurch gekennzeichnet, daß** die Gesamtmenge an einer oder mehreren erfindungsgemäß verwendeten grenzflächenaktiven Acrylat-Alkylacrylat-Copolymeren in den fertigen kosmetischen oder dermatologischen Zubereitungen aus dem Bereich von 0,1 -10,0 Gew.-%, bevorzugt 0,5 - 2,5 Gew.-% gewählt wird, bezogen auf das Gesamtgewicht der Zubereitungen.

7. Zubereitungen nach Anspruch 1, **dadurch gekennzeichnet, daß** die Gesamtmenge an einer oder mehreren erfindungsgemäß verwendeten Alkoholen in den fertigen kosmetischen oder dermatologischen Zubereitungen aus dem Bereich von 0,1 - 25,0 Gew.-%, bevorzugt 0,5 - 15,0 Gew.-% gewählt wird, bezogen auf das Gesamtgewicht der Zubereitungen.

8. Zubereitungen nach Anspruch 1, **dadurch gekennzeichnet, daß** sie in Form von Gelen vorliegen.

9. Zubereitungen nach Anspruch 8, **dadurch gekennzeichnet, daß** gasförmige, feste und oder flüssige Objekte in die Gele eingebettet vorliegen.

## Claims

1. Cosmetic and dermatological detersive preparations comprising:
(a) an effective amount of one or more surface-active substances selected from the group consisting of alkyl glucosides, which have the structural formula 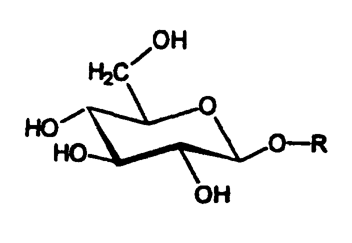 where R is a branched or unbranched alkyl radical having from 1 to 24 carbon atoms,
(b) an effective amount of one or more gel-forming acrylate-alkyl acrylate copolymers and
(c) branched and/or unbranched aliphatic alcohols having 2 - 6 carbon atoms and one or more OH functions.

2. Preparations according to Claim 1, **characterized in that** R in the structural formula of the alkyl glucoside is selected from the group consisting of unbranched alkyl radicals, the hexyl, heptyl, octyl, nonyl, decyl, undecyl, dodecyl and tetradecyl radicals being preferred.

3. Preparations according to Claim 1, **characterized in that** the total amount of one or more surface-active alkyl glucosides used according to the invention in the finished cosmetic or dermatological preparations is selected from the range 0.1 - 25.0% by weight, preferably 0.5 - 15.0% by weight, based on the total weight of the preparations.

4. Preparations according to Claim 1, **characterized in that** the acrylate-alkyl acrylate copolymer(s) are selected from the group consisting of so-called carbomers, in particular from the group consisting of carbomers having the structure: 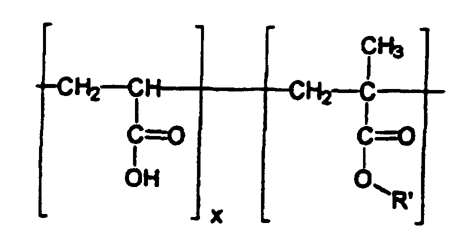 where R' is a long-chain alkyl radical, and x and y are numbers which symbolize the respective stoichiometric amount of each of the comonomers.

5. Preparations according to Claim 1, **characterized in that** the alcohols are selected from the group consisting of ethylene glycol, propylene glycol, glycerol, isopropyl alcohol, 1,3-butylene glycol, 2,3-butylene glycol and ethanol.

6. Preparations according to Claim 1, **characterized in that** the total amount of one or more surface-active acrylate-alkyl acrylate copolymers used according to the invention in the finished cosmetic or dermatological preparations is selected from the range 0.1 - 10.0% by weight, preferably 0.5 - 2.5% by weight, based on the total weight of the preparations.

7. Preparations according to Claim 1, **characterized in that** the total amount of one or more alcohols used according to the invention in the finished cosmetic or dermatological preparations is selected from the range 0.1 - 25.0% by weight, preferably 0.5 - 15.0% by weight, based on the total weight of the preparations.

8. Preparations according to Claim 1, **characterized in that** they are in the form of gels.

9. Preparations according to Claim 8, **characterized in that** gaseous, solid and [lacuna] or liquid objects are embedded in the gels.

## Revendications

1. Préparations cosmétiques et dermatologiques lavantes, contenant :
(a) une quantité efficace d'une ou plusieurs substances tensioactives choisies dans le groupe des alkylglucosides qui se distinguent par la formule développée 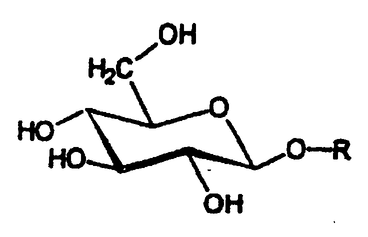 dans laquelle R représente un radical alkyle ramifié ou non ramifié ayant de 1 à 24 atomes de carbone,
(b) une quantité efficace d'un ou plusieurs copolymères acrylate/alkylacrylate et
(c) des alcools aliphatiques ramifiés et/ou non ramifiés ayant de 2 à 6 atomes de carbone et comportant une ou plusieurs fonctions OH.

2. Préparations selon la revendication 1, **caractérisées en ce que** R dans la formule développée de l'alkylglucoside est choisi dans le groupe des radicaux alkyle non ramifiés, les radicaux hexyle, heptyle, octyle, nonyle, décyle, undécyle, dodécyle et tétradécyle étant préférés.

3. Préparations selon la revendication 1, **caractérisées en ce que** dans les préparations cosmétiques ou dermatologiques finales, la quantité totale d'un ou plusieurs alkylglucosides tensioactifs utilisés selon l'invention se situe avantageusement dans la plage allant de 0,1 à 25,0 % en poids, de préférence de 0,5 à 15,0 % en poids, par rapport au poids total des préparations.

4. Préparations selon la revendication 1, **caractérisées en ce que** le ou les copolymères acrylate/alkylacrylate est(sont) choisi(s) dans le groupe des dénommés carbomères, en particulier choisi(s) dans le groupe des carbomères de formule: 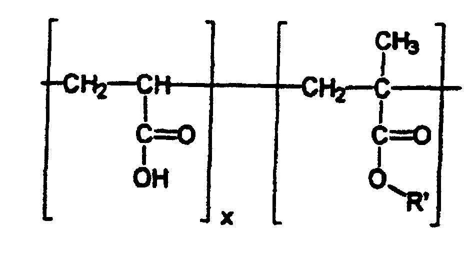 dans laquelle R' représente un radical alkyle à longue chaîne, x et y représentent des nombres qui indiquent la proportion stoechiométrique des comonomères respectifs.

5. Préparations selon la revendication 1, **caractérisées en ce que** les alcools sont choisis dans le groupe constitué par l'éthylèneglycol, le propylèneglycol, le glycérol, l'alcool isopropylique, le 1,3-butylèneglycol, le 2,3-butylèneglycol, l'éthanol.

6. Préparations selon la revendication 1, **caractérisées en ce que** la quantité totale d'un ou plusieurs copolymères acrylate/alkylacrylate tensioactifs utilisés selon l'invention dans les préparations cosmétiques ou dermatologiques finales se situe dans la plage allant de 0,1 à 10,0 % en poids, de préférence de 0,5 à 2,5 % en poids, par rapport au poids total des préparations.

7. Préparations selon la revendication 1, **caractérisées en ce que** la quantité totale d'un ou plusieurs alcools utilisés selon l'invention dans les préparations cosmétiques ou dermatologiques finales est choisie dans la plage allant de 0,1 à 25,0 % en poids, de préférence de 0,5 à 15,0 % en poids, par rapport au poids total des préparations.

8. Préparations selon la revendication 1, **caractérisées en ce qu'**elles se trouvent sous forme de gels.

9. Préparations selon la revendication 8, **caractérisées en ce que** des objets gazeux, solides et/ou liquides se trouvent inclus dans les gels.
